# EUROPEAN PATENT APPLICATION

(11) **EP 3 199 163 A1**
(43) Date of publication of application: **02.08.2017**
(21) Application number: 15845327.4
(22) Date of filing: 23.09.2015
(51) Int. Cl.: A61K 31/65, A61K 9/127, A61P 27/02

(54) **COMPOSITION OF DOXYCYCLINE IN LIPOSOMES FOR THE PREVENTION, IMPROVEMENT AND/OR TREATMENT OF OCULAR PATHOLOGIES**

(30) Priority: 23.09.2014 ES 201431379; 16.12.2014 ES 201431854
(71) Applicant: Agencia Pública Empresarial Sanitaria Hospital Alto Guadalquivir, 23740 Andújar (Jaén) (ES)
(72) Inventor: VILLEGAS BECERRIL, Enrique, 23740 Andújar, Jaén (ES)
(74) Representative: Hoffmann Eitle
(86) International application number: PCT/ES2015/070695
(87) International publication number: WO 2016/046442

(57) **Abstract**

The invention relates to use of a composition comprising doxycycline in the production of a topically administered medicament for the prevention, improvement and/or treatment of ocular disorders.

## Description

### FIELD OF THE INVENTION

The present invention is comprised in the field of biology and medicine and relates to the use of doxycycline for the prevention, improvement and/or treatment of ocular disorders. Preferably, the present invention relates to the topical use of doxycycline in liposomes in the form of stable eye drops for the prevention, improvement and/or treatment of blepharitis, microthrombosis in the anterior pole and chorioretinal vascular disorders.

### BACKGROUND OF THE INVENTION

Blepharitis is one of the most common ophthalmological diseases. It represents a wide range of acute and chronic diseases characterized by inflammation of the free margin of the eyelid. For both patients and ophthalmologists, this disease is very hard to understand, frustrating due to its chronic nature and extremely tedious as a result of the prolonged treatment it requires.

The free margin of the eyelid is the source of inflammation which spreads to the cornea and bulbar conjunctiva. This means that different types of patients are observed: patients with bulbar conjunctival vessel congestion, with infiltration of the cornea and conjunctiva adnexa and pannus (vessel invasion into the peripheral cornea), particularly in the inferior cornea.

It is important to know the different manifestations of blepharitis in order to apply suitable treatment (Raskin et al., 1992. Infect. Dis. Clin. North Am. 6(4), 777-787).

More than 80 agents responsible for blepharitis have been identified (Ostler, 1993. Diseases of the external eye and adnexa, Baltimore, Williiams and Wilkins*),* the most common ones are *Streptococus, Staphilococus, Pseudomonas, Moraxella* and *Actinomices,* in addition to other not so common ones such as Anthrax, *Pasteurella, Clostridium* and *Micobasterium tuberculosis* (Groden et al., 1991. Cornea 10(1), 50-53). There are also other agents of viral origin (*Molluscum contagiosum, Herpes simplex* and *Herpes zoster)* and fungi *(Coccidioides, Microsporum blastomices, Candida* and *Aspergillus).* Furthermore, there are also allergic disorders (atopy and contact dermatitis) and other Steven-Johnson-type syndromes, ichthyosis or Lyell's syndrome.

There are currently four blepharitis treatment alternatives: eyelid hygiene, topical antibiotics, systemic antibiotics such as tetracyclines (Neiberg and Sowka, 2008. Optometry 79(3), 133-137) and corticosteroids.

Keeping the free margin of the eyelid clean is fundamental for eliminating growing pathogens. Oral tetracyclines are a fundamental complementary treatment to ease the symptoms of blepharitis (Dougherty et al., 1991. Invest. Ophthalmol. Vis. Sci. 132(11), 2970-2975). Oral tetracyclines are also used in certain apical corneal barrier disruptions (De Paiva et al., 2006. Invest. Ophthalmol. Vis. Sci. 47(7), 2847-56) and in other pathologies of the anterior pole (Pinna et al., 1999. Br. J. Ophthalmol. 83(7), 771-773). Doxycycline is an antibiotic belonging to the tetracycline family which is not usually used topically. De Paiva *et al.* have already used topical doxycycline for the treatment of dry eye, although it has never been used for blepharitis treatment.

Doxycycline has anti-infection effect and anti-inflammatory effect (Wang et al., 2008. Clin. Experiment. Ophthalmol. 36(1), 8-12; Dursun et al., 2008. Am. J. Ophthalmol. 132(1), 8-13). The amount of orally administered tetracycline may not reach sufficient concentration in the tear film where it exerts its function (Smith et al., 2008. Br. J. Ophthalmol. 92(6), 856-859).

Doxycycline has a protective effect on the walls of the microvasculature. In fact, it is used in the prevention of aortic aneurysms through the inhibition of enzymes degrading vessel walls. In certain groups of hemodialysis patients, doxycycline proved to be effective in the prevention of thrombosis (Diskin et al., 1998. Nephron 78, 365-368; Saran et al., 2002. Am. J. Kidney Dis. 40, 1255-1263).

The protective effect of doxycycline on the vessel wall seems to be determined by metalloproteinase matrix activity inhibition (Sorsa et al., 1994. Ann. NY. Acad. Sci. 732, 375-378).

In a study conducted on rats, doxycycline was capable of preventing aneurysmal dilatation without subsequent signs of inflammation (Kaito et al., 2003. Surg. Today 33, 426-433). In other work, patients taking doxycycline were found to have less vascular intimal layer proliferation as well as less muscle cell migration (Islam et al., 2003. Am. J. Pathol. 163, 1557-1566).

The physiopathology of diabetic retinopathy (DR) has yet to be fully clarified, but most authors attribute it to vascular changes. In contrast, it has been recently discovered that there are changes in the glia and on a neuronal level that may explain part of this physiopathology. (Antonetti et al., 2012. N. Engl. J. Med. 366(13), 1227-1239). Chronic inflammatory changes occurring in DR in experimental models include increased local nitric oxide production, increased intracellular molecular adhesion, leukostasis and increased cell expression by means of cytokines which are in turn associated with tissue damage and neuronal damage and loss (Adamis et al., 2008. Semin. Immunopathol. 30(2), 65-84; Tang et al., 2011. Prog. Retin. Eye Res. 30(5), 343-358).

The microglia represents the first line of the immune system against attacks and usually remains inactive, but in situations such as DR it is activated, inducing inflammatory changes (Rungger-Brändle et al., 2000. Invest. Ophthalmol. Vis Sci. 41(7), 1971-1980; Zeng et al., 2000. Vis Neurosci. 17(3), 463-471; Zeng et al., 2008. Arch. Ophthalmol. 126(2), 227-232; Barber et al., 2005. Invest Ophthalmol Vis Sci. 46(6), 2210-2218; Gaucher et al., 2007. Vision Res. 47(5), 612-623; Ibrahim et al., 2007. Diabetes 60(4), 1122-1133; Krady et al., 2005. Diabetes 54(5), 1559-1565; Vincent et al., 2007. Diabetes 56(1):224-230).

Tetracycline and its derivatives have been proven to have an anti-inflammatory effect and neuroprotective effects, in addition to anti-bacterial effects, in cultures and in animal models. With respect to DR, some studies have proven that doxycycline at low doses inhibits microglia inflammatory changes, metalloproteinase activity and cell apoptosis (Wang et al., 2005. Neurochem Int. 47(1-2), 152-158; Federici, 2011. Pharmacol. Res. 64(6), 614-623; Baptiste et al., 2005. Neuroscience 134(2), 575-582; Griffin et al., 2011. Pharmacol. Res. 63(2), 102-107).

In a clinical trial including 5 patients with foveal involvement due to diabetic macular edema who were treated with 100 mg oral minocycline 2 times a day for 6 months, visual acuity of said patients improved and macular thickness dropped significantly compared with historic controls from earlier studies (Cukras et al., 2012. Invest. Ophthalmol. Vis. Sci. 53(7), 3865-3874).

In summary, topical doxycycline may be minimally invasive for the organism, lacking unwanted side effects such as damage to the gastrointestinal tract, and its protective effects on the microvasculature may delay the onset of DR.

Like other tetracyclines, doxycycline is an antibiotic that has a stability problem. It loses microbial activity as it is broken down and may even become toxic. Liposomes could be a way to integrate doxycycline, which increases its stability.

### BRIEF DESCRIPTION OF THE INVENTION

The author of the present invention has developed and verified that a topical doxycycline composition could be useful for the treatment of blepharitis, disorders of the anterior surface of the eye involving infection and/or inflammation. The topical route would prevent the side effects caused by oral administration.

Other applications in addition to those already described could be:
- The prophylaxis of intraoperative infections such as endophthalmitis in blepharitis patients.
- Dry eye, ocular rosacea.
- Use in emergencies for traumas and ulcerations.
- For the treatment, prevention and to avoid progression of diabetic retinopathy.

Therefore, a **first aspect** of the invention relates to the use of a composition, hereinafter composition of the invention, comprising doxycycline or any of its pharmaceutically acceptable salts, esters, tautomers, solvates and hydrates, or any combinations thereof, in the production of a medicament for the prevention, improvement and/or treatment of ocular pathologies. Alternatively, it relates to a composition comprising doxycycline or any of its pharmaceutically acceptable salts, esters, tautomers, solvates and hydrates, or any combinations thereof, for use in the prevention, improvement and/or treatment of ocular pathologies.

In a preferred embodiment of this aspect of the invention, the composition is a composition for topical administration. In another preferred embodiment, the ocular pathology is selected from: blepharitis, microthrombosis in the anterior pole and chorioretinal vascular disorders, or any combinations thereof.

In another preferred embodiment of the invention, the ocular pathology is diabetic retinopathy.

In another preferred embodiment, the doxycycline is included in liposomes. More preferably, the liposomes have a cationic character, and even more preferably, a neutral character.

In another preferred embodiment, the composition of the invention has a concentration between 0.5 and 50% of liposomes, more preferably between 1 and 25%, more preferably between 2 and 10%, even more preferably between 4 and 6%, and even much more preferably 5% of liposomes.

In another preferred embodiment, the liposomes are composed of lecithin with 50 to 99% of phosphatidylcholine, more preferably with 70 to 95% of phosphatidylcholine, and even more preferably with about 90% of phosphatidylcholine.

In another preferred embodiment, the liposomes are stabilized in an ionic solution, and more preferably in phosphate buffer. In another more preferred embodiment of this aspect of the invention, the pH of the phosphate buffer is adjusted to a value between 4 and 10, more preferably between 5 and 9, and even more preferably between 6 and 8.

In another preferred embodiment, the liposomes are stabilized in a non-ionic solution. More preferably, the non-ionic solution is selected from a mannitol solution and a glucose solution, and it is more preferably a mannitol solution. Even more preferably, the mannitol solution has a concentration between 1 and 20%, preferably between 2.5 and 10%, and more preferably about 5% mannitol. Even much more preferably, the pH of the solution is adjusted to a value between 3 and 9, more preferably between 4 and 8, and even more preferably between 5 and 7.

In another preferred embodiment, the liposomes have a final size between 20 and 1000 nm, more preferably between 50 and 500 nm, even more preferably between 100 and 300 nm, and even much more preferably about 200 nm.

In another preferred embodiment, the composition has a doxycycline concentration between 0.01 and 5%, more preferably between 0.025 and 1%, even more preferably between 0.04 and 0.06%, and even much more preferably about 0.05%.

In another preferred embodiment, the composition is a pharmaceutical composition. More preferably, the composition further comprises a pharmaceutically acceptable vehicle. Even more preferably, the composition further comprises another active ingredient.

In another preferred embodiment, the liposomes comprise antioxidants. More preferably, they further comprise antioxidants.

In another preferred embodiment of this aspect of the invention, the composition is sterilized.

A **second aspect** of the invention relates to a dosage form, hereinafter dosage form of the invention, comprising the composition of the invention.

In a preferred embodiment of this aspect, the dosage form of the invention is selected from the list consisting of: dressing, ointment, paste, cream, solution, suspension, emulsion, lotion, liniment, jelly, gel, foam, powder, or any combinations thereof. More preferably, it is a solution, and even more preferably an ophthalmic solution.

A **third aspect** of the invention relates to the use of the dosage form of the invention in the production of a medicament for the prevention, improvement and/or treatment of ocular pathologies. Alternatively, it relates to the dosage form of the invention for use in the prevention, improvement and/or treatment of ocular pathologies.

In a preferred embodiment of this aspect of the invention, the composition is a composition for topical administration. In another preferred embodiment, the ocular pathology is selected from: blepharitis, microthrombosis in the anterior pole and chorioretinal vascular disorders, or any combinations thereof. In another preferred embodiment of the invention, the ocular pathology is diabetic retinopathy.

### DESCRIPTION OF THE DRAWINGS

**Figure 1****.** Ocular dryness test record obtained by means of Schirmer's test in which it can be seen that after treatment, moisture in the eye increased as a result of this treatment.
**Figure 2****.** Individual analysis of each patient according to dry eye questionnaire score. All patients completed a questionnaire to evaluate the symptoms derived from blepharitis-dry eye like disease. In all the cases, symptoms improved after treatment with doxycycline with a significant drop in said score.
**Figure 3****.** Overall results with the sum of all the scores in all patients in the dry eye questionnaire. Symptoms decreased significantly after treatment with doxycycline. This figure complements Figure 2.
**Figure 4****.** Record of the patient's degrees of hyperemia (red eye, redness of the eye) before and after treatment with topical doxycycline according to the Oxford scale. In all cases, this hyperemia was considerably less after this treatment.

### DETAILED DESCRIPTION OF THE INVENTION

The authors of the present invention have developed a doxycycline composition in liposomes with anti-infection and anti-inflammatory effects for topical use.

Therefore, a **first aspect** of the present invention relates to the use of a composition comprising doxycycline or any of its pharmaceutically acceptable salts, esters, tautomers, polymorphs, analogs, solvates and hydrates, or any combinations thereof, in the production of a medicament for the prevention, improvement and/or treatment of ocular pathologies. Alternatively, it relates to a composition comprising doxycycline or any of its pharmaceutically acceptable salts, esters, tautomers, polymorphs, analogs, solvates and hydrates, or any combinations thereof, for use in the prevention, improvement and/or treatment of ocular pathologies.

Preferably, the administration of the composition of the invention is by topical route.

In this specification, doxycycline is understood as an antibiotic from the tetracycline group having the chemical formula C₂₂H₂₄N₂O₈. It is a semi-synthetic tetracycline-related derivative. It has a broad-spectrum bacteriostatic effect and acts by inhibiting protein synthesis, blocking the binding of transfer RNA to the ribosomal complex. Specifically, binding occurs in the 30S ribosomal subunit of sensitive organisms. Furthermore, it has anti-inflammatory action, acting on polynuclear neutrophils involved in the inflammatory stage of specific infections. This antibiotic prevents growth and propagation of both Gram positive and Gram negative bacteria. It has the following **formula (I):**

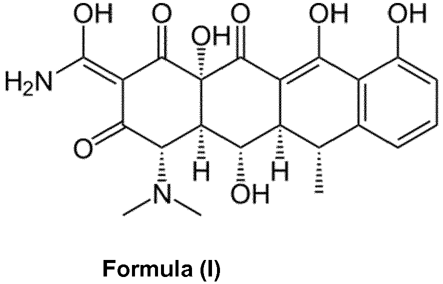

The IUPAC nomenclature is (2Z,4S,4aR,5S,5aR,6R,12aS)-2-(amino-hydroxymethylidene)-4-dimethylamino-5,10,11,12a-tetrahydroxy-6-methyl-4a,5,5a,6-tetrahydro-4H-tetracene-1,3,12-trione and the CAS number is 564-25-0.

The compounds of the present invention represented by formula (I) can include isomers, depending on the presence of multiple bonds, including optical isomers or enantiomers, depending on the presence of chiral centers. Individual isomers, enantiomers or diastereoisomers and mixtures thereof fall within the scope of the present invention, i.e., the term isomer also refers to any mixture of isomers, such as diastereomeric isomers, racemic isomers, etc., even the optically active isomers thereof or mixtures thereof in different proportions. Individual enantiomers or diastereoisomers, as well as mixtures thereof, can be separated by means of conventional techniques.

Likewise, the scope of this invention includes the prodrugs of the compounds of formula (I). As it is used herein, the term *"prodrug"* includes any derivative of a compound of formula (I), for example and in a non-limiting manner: esters (including carboxylic acids esters, amino acid esters, phosphate esters, sulfonate esters of metal salts, etc.), carbamates, amides, etc., which, when administered to an individual, can be transformed directly or indirectly into said compound of formula (I) in the mentioned individual. Advantageously, said derivative is a compound that increases the bioavailability of the compound of formula (I) when administered to an individual or enhances the release of the compound of formula (I) in a biological compartment. The nature of said derivative is not critical provided that it can be administered to an individual and it provides the compound of formula (I) in a biological compartment of an individual. Said prodrug can be prepared by means of conventional methods known by those skilled in the art.

As it is used herein, the term "derivative" includes both pharmaceutically acceptable compounds, i.e., derivatives of the compound of formula (I) which can be used in the production of a medicament or food compositions, and non-pharmaceutically acceptable derivatives since they may be useful in the preparation of pharmaceutically acceptable derivatives.

The compounds of the invention can be in crystalline form as free compounds or solvates. In this sense, as it is used herein the term "solvate" includes both pharmaceutically acceptable solvates, i.e., solvates of the compound of formula (I) which can be used in the production of a medicament, and non-pharmaceutically acceptable solvates which may be useful in the preparation of pharmaceutically acceptable solvates or salts. The nature of the pharmaceutically acceptable solvate is not critical provided that it is pharmaceutically acceptable. In a particular embodiment, the solvate is a hydrate. The solvates can be obtained by conventional solvation methods known by those skilled in the art.

For application in therapy, the compounds of formula (I), its salts, prodrugs or solvates, will preferably be in a pharmaceutically acceptable or substantially pure form, i.e., having a pharmaceutically acceptable level of purity excluding normal pharmaceutical additives such as diluents and carriers, and not including material considered toxic at normal dosage levels. The levels of purity for the active ingredient are preferably greater than 50%, more preferably greater than 70%, and even more preferably greater than 90%. In a preferred embodiment, they are greater than 95% of the compound of formula (I) or of its salts, solvates or prodrugs.

In another preferred embodiment, the ocular pathology is selected from: blepharitis, microthrombosis in the anterior pole and chorioretinal vascular disorders, or any combinations thereof. In another preferred embodiment of the invention, the ocular pathology is diabetic retinopathy.

In this specification, blepharitis is understood to be inflammation of the tissue forming the eyelid. It usually originates from an irregular working of the glands in the eyelid margin that leads to irritation and scab formation, which provides optimal conditions for the growth of bacteria that further irritate the eyelid and aggravate the process. If the process is not stopped, progressive worsening will occur with painful inflammation of the eyelid margin, extreme discomfort on the ocular surface and even decreased vision. Blepharitis can be of two types:
- Squamous blepharitis: patients present scaly eyelids due to its appearance and it is characterized by the formation of scales between eyelashes that often fall into the eyes, causing the feeling of there being something in the eyes. The eyes are red. The margins of the eyelids are red most of the time, giving the impression that the patient has been crying, and it can cause local itching. It is the cause of local bacterial and fungal infection on seborrheic skin.
- Allergic blepharitis: It is a form of manifestation of a contact allergy, mostly to cosmetic products, for example eyeshadows, eye pencils and blushes, as well as cosmetic solvents for removing said products. However, actually, any substance which contaminates the fingers and is brought to the eyes by means of the hands can cause acute or chronic dermatitis of the eyelids, such as plants and their saps, vegetables, acrylic plastics or even medicaments for the legs or feet, to mention a few examples.

In this specification, microthrombosis of the anterior pole is understood as the formation of thrombi in capillaries and vessels of small caliber located in the anterior part of the eyeball: conjunctiva, cornea and lens.

In this specification, chorioretinal vascular disorder is understood as that situation in which blood does not circulate the way it should due to any possible event in the chorioretinal area.

In this specification, diabetic retinopathy is understood as an ocular diabetes complication which is caused by the deterioration of blood vessels irrigating the retina. Damage to retinal blood vessels can result in the blood vessels experiencing a leakage of fluid or blood. If the disease progresses, new blood vessels form and the fibrous tissue proliferates in the retina, which results in deteriorating vision since the image sent to the brain becomes blurred.

In a preferred embodiment of this aspect of the invention, the doxycycline is included in liposomes.

In this specification, liposomes are understood to be spherical vesicles with a phospholipid membrane. The arrangement of phospholipids in liposome formation causes both the inside and outside to be water-soluble, whereas the inside is fat-soluble. Use thereof as transporters for transporting substances between the exterior and the interior of a cell has been described, hence the interest thereof in biotechnology. Liposomes are usually produced from lecithin obtained from soy, egg yolk and brain tissue, although other lipids are added to increase their stability. Liposome size usually ranges between 20 nm and 500 µm and liposomes can be classified by size: liposomes ranging from 20 to 70 nm are referred to as Small Unilamellar Vesicles (SUVs), liposomes ranging from 70 to 400 nm are referred to as Large Unilamellar Vesicles (LUVs). It is also possible to form a special type of liposomes referred to as Giant Unilamellar Vesicles (GUVs) having a size far larger than the foregoing and a diameter ranging between about 10 and 100 µm. On the other hand, there are also Oligolamellar Vesicles (OLVs) which have a few superimposed layers, and Multilamellar Vesicles (MLVs) which have many superimposed layers. These liposomes have highly variable size distributions and they can range from about 500 nm to 2000 nm in the same sample.

In a preferred embodiment of this aspect of the invention, the liposomes have a cationic character. In another preferred embodiment of this aspect of the invention, the cationic character is achieved by producing liposomes with physiological saline.

In the present specification, physiological saline or physiological saline solution is understood as an aqueous solution compatible with living organisms as a result of its defined osmotic, pH and ionic strength characteristics. It usually consists of water, electrolytes, and additionally other substances such as glucose. Its biochemical composition is 0.9% NaCl.

In another preferred embodiment of this aspect of the invention, the liposomes have a neutral character. In another preferred embodiment of this aspect of the invention, the neutral character is achieved by producing liposomes with purified water.

In the present specification, purified water is understood as water improved or enriched in a laboratory with an agent. Purified water is water treated for a specific use in science or engineering. Three types of purified water can be distinguished: distilled, double-distilled and deionized.

In a preferred embodiment of this aspect of the invention, the composition has a concentration between 0.5 and 50% of liposomes, more preferably between 1 and 25%, more preferably between 2 and 10%, more preferably between 4 and 6%, and even more preferably about 5%.

In a preferred embodiment of this aspect of the invention, the liposomes are composed of lecithin with 50-99% of phosphatidylcholine, more preferably with 70-95% of phosphatidylcholine, and even more preferably with about 90% of phosphatidylcholine.

Phosphatidylcholine is a phospholipid and usually the major component of lipid bilayers. Phospholipid is made up of a choline head group and glycerophosphoric acid, with a variety of fatty acids, one of them being a saturated fatty acid and one of them an unsaturated fatty acid. Phospholipase D catalyzes phosphatidylcholine hydrolysis to form phosphatidic acid, releasing the cytosol-soluble choline head group. However, phosphatidylcholine is a neutral lipid with an electric dipole moment of ∼ 10 D.

In a preferred embodiment of this aspect of the invention, the liposomes are stabilized in an ionic solution, preferably phosphate buffer. In another preferred embodiment of this aspect of the invention, the pH of the phosphate buffer is adjusted between 4 and 10, more preferably between 5 and 9, and even more preferably between 6 and 8.

In a preferred embodiment of this aspect of the invention, the liposomes are stabilized in a non-ionic solution. In a preferred embodiment of this aspect of the invention, the non-ionic solution is preferably selected from a mannitol solution and a glucose solution, preferably a mannitol solution. In a preferred embodiment of this aspect of the invention, the mannitol solution is 1-20%, preferably 2.5-10%, and more preferably about 5%. In another preferred embodiment of this aspect of the invention, the pH of the mannitol solution is adjusted between 3 and 9, more preferably between 4 and 8, and even more preferably between 5 and 7.

In a preferred embodiment of this aspect of the invention, the liposomes have a final size between 20 and 1000 nm, more preferably between 50 and 500 nm, even more preferably between 100 and 300 nm, and even more preferably about 200 nm.

In a preferred embodiment of this aspect of the invention, the liposomes have a doxycycline concentration between 0.01 and 5%, more preferably between 0.025 and 1%, more preferably 0.04-0.06%, and even more preferably about 0.05%.

In a preferred embodiment of this aspect of the invention, the composition is a pharmaceutical composition.

In a preferred embodiment of this aspect of the invention, the composition further comprises a pharmaceutically acceptable vehicle.

In a preferred embodiment of this aspect of the invention, the composition further comprises another active ingredient.

As it is used herein, the term *"active ingredient", "active substance",* "pharmaceutically active *substance",* or *"pharmaceutically active ingredient"* means any component that may provide a pharmacological activity or another different effect in the diagnosis, cure, mitigation, treatment, or prevention of a disease, or that affects the body structure or function of humans or other animals. The term includes those components that promote a chemical change in the production of the drug and are present in the drug in an envisaged modified form, providing the specific activity or effect.

In a preferred embodiment of this aspect of the invention, the liposomes contain antioxidants.

In a preferred embodiment of this aspect of the invention, the liposomes contain stabilizers.

In a preferred embodiment of this aspect of the invention, the composition is sterilized. The sterilization process for sterilizing the composition can be carried out by any sterilization method known in the state of the art. In a preferred embodiment of this aspect of the invention, sterilization is achieved by filtration.

The compositions of the present invention can be formulated for administration to an animal, and more preferably to a mammal, including humans, in a variety of forms known in the state of the art. In this sense, the compositions can be, without limitation, in a sterile aqueous solution or in biological fluids, such as serum. The aqueous solutions may or may not be buffered and they have additional active or inactive components. The additional components include salts for modulating ionic strength, preservatives and other substances including, but not limited to, antimicrobial agents, antioxidants, chelating agents, and the like, and nutrients including glucose, dextrose, vitamins and minerals. Alternatively, the compositions can be prepared for administration in solid form. The compositions can be combined with several inert vehicles or excipients, including but not limited to agglutinants such as microcrystalline cellulose, tragacanth gum, or gelatin; excipients such as starch or lactose; dispersing agents such as alginic acid or corn starch; lubricants such as magnesium stearate, glidants such as colloidal silicon dioxide; sweetening agents such as sucrose or saccharin; or aromatizing agents such as mint or methyl salicylate.

A **second aspect** of the invention relates to a dosage form, hereinafter dosage form of the invention, comprising the composition according to the first aspect of the invention.

In this specification, "dosage form" is understood as the mixture of one or more active ingredients with or without additives having physical characteristics for suitable dosing, conservation, administration and bioavailability.

In a preferred embodiment of this aspect of the invention, the dosage form is selected from the list comprising: dressing, ointment, paste, cream, solution, suspension, emulsion, lotion, liniment, jelly, gel, foam, powder, or any combinations thereof.

A "dressing" or "patch" is a dosage form consisting of a solid or semisolid form containing the active ingredient or ingredients and additives, spread on a piece of fabric, plastic or adhesive tape, acting as a support and protection, in addition to having an occlusive effect and macerating action which in addition allows it to come into direct contact with skin and to soften with body temperature.

An "unguent" or "ointment" is a dosage form consisting of a preparation having a soft consistency containing the active ingredient or ingredients and additives incorporated in a suitable base giving it body and consistency. It is adhered and applied to the skin and mucous membranes. This base can be fat- or water-soluble, generally anhydrous or with a maximum of 20% water. It is also referred to as hydrophilic unguent when it contains a base that can be washed away or removed with water.

A "paste" is a dosage form consisting of a semisolid form containing the active ingredient or ingredients and additives, made based on a high concentration of insoluble powders (20 to 50%), in weak abrasive or absorbent fatty or aqueous bases combined with soaps.

A "cream" is a dosage form consisting of a liquid or semisolid preparation containing the active ingredient or ingredients and additives required for obtaining an emulsion, generally an oil-in-water emulsion, with a water content greater than 20%.

A "solution" is a dosage form consisting of a transparent and homogenous liquid preparation obtained by dissolving the active ingredient or ingredients and additives in water and is intended for external or internal use. In the case of injectable solutions for the eyes and ears, they must be sterile. The term "solution" includes solutions.

A "suspension" is a dosage form consisting of a dispersed system formed by two phases which contain the active ingredient or ingredients and additives. One of the phases, the continuous or external phase, is generally a liquid or a semisolid, and the dispersed or internal phase is made up of solids (active ingredients) that are insoluble but dispersible in the external phase. In the case of injectable suspensions, they must be sterile.

An "emulsion" is a dosage form consisting of a heterogeneous system generally made up of two liquids not miscible with one another, in which the dispersed phase is formed by small globules distributed in the vehicle in which they are immiscible. The dispersed phase is also known as an internal phase and the dispersion medium is known as an external or continuous phase. There are water-in-oil-type or oil-in-water-type emulsions and they can be presented as semisolids or liquids. The active ingredient or ingredients and additives can be in the external or internal phase.

A "lotion" is a dosage form that can be presented as a solution, suspension or emulsion, containing the active ingredient or ingredients and additives, and the dispersing agent of which is predominantly water.

A "liniment" is a dosage form consisting of an emulsion, solution or liquid presentation containing the active ingredient or ingredients and additives the vehicle of which is an aqueous, alcoholic or oily vehicle.

A "jelly" is a dosage form consisting of a semisolid colloid containing the active ingredient or ingredients and additives, the water-soluble base of which is generally made up of gums such as tragacanth gum, other bases used are: glycerin, pectin, alginates, boroglycerine compounds, synthetic derivatives or natural substances such as carboxymethyl cellulose.

A "gel" is a dosage form consisting of semisolid preparation containing the active ingredient or ingredients and additives, solids in a liquid which can be water, alcohol or oil, such that a network of particles trapped in the liquid phase is formed.

A "foam" is a dosage form consisting of a semisolid preparation formed by two phases: a liquid phase carrying the active ingredient or ingredients and additives, and another gaseous phase carrying propellant gas for the product to come out in the form of a cloud.

"Powder" is a dosage form consisting of a solid form containing the active ingredient or ingredients and additives that are finely grounded and mixed to assure homogeneity.

In a preferred embodiment of this aspect of the invention, the dosage form is a solution, preferably an ophthalmic solution or eye drops.

A **third aspect** of the invention relates to the use of the dosage form of the invention in the production of a medicament for the prevention, improvement and/or treatment of ocular pathologies. Alternatively, the invention relates to the dosage form of the invention for use in the prevention, improvement and/or treatment of ocular pathologies.

In a preferred embodiment of this aspect of the invention, the composition is a composition for topical administration. In another preferred embodiment, the ocular pathology is selected from: blepharitis, microthrombosis in the anterior pole and chorioretinal vascular disorders, or any combinations thereof.

As it is used herein, the term "medicament" refers to any substance used for the prevention, diagnosis, relief, treatment or cure of diseases in humans and animals. In the context of the present invention, the disease is an ocular disease, preferably blepharitis, microthrombosis in the anterior pole and/or chorioretinal vascular disorders.

Throughout the description and claims the word "comprises" and variants thereof do not seek to exclude other technical features, additions, components or steps. For those skilled in the art, other objects, advantages and features of the invention will be inferred in part from the description and in part from putting the invention into practice. The following examples and drawings are provided by way of illustration and do not seek to limit the present invention.

### EXAMPLES OF THE INVENTION

### Example 1: Experimental study with eye drops containing 0.05% doxycycline in blepharitis

### MATERIAL AND METHODS

The authors of the present invention proposed to first evaluate experience with the use of doxycycline in blepharitis before designing a clinical trial. The evaluation included 12 patients with this disease whose Cornea and Contact Lens Research Lab hyperemia score was maximum (4) and in whom signs of staphylococcal infection were present (collarettes and telangiectasias) and the disease was confirmed due to this bacterium.

### RESULTS

### Conjunctival culture

The twelve patients showed a positive staphylococcus culture before treatment with doxycycline. After treatment with these 0.05% eye drops for three weeks, said culture was repeated; none of the twelve patients showed a positive staphylococcus culture at that time.

### Schirmer's test

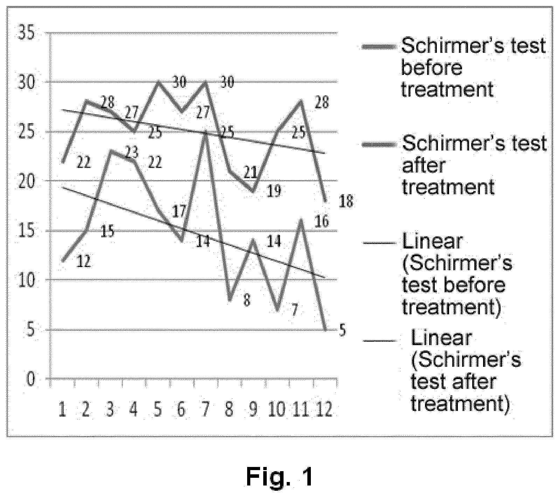

This test is used to assess dry eye status in blepharitis (in millimeters). As can be confirmed - though even in the absence of validated statistical tests, all patients showed a baseline tear secretion lower than that shown after treatment. This leads the authors to think of the possibility of using doxycycline against this dry syndrome (Figure 1).

### Symptom score according to the dry eye questionnaire

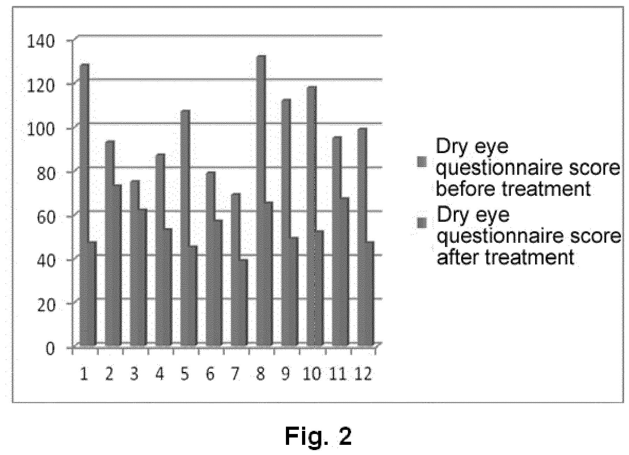

This test was designed (see test design literature) for assessing the symptoms the patient experiences and they can be expressed by means of a score based on questions about these symptoms. It can be seen how each and every one of the patients has a higher score before treatment, which indicates that all the patients of the sample had at least moderate symptoms, most of them being severe. After applying eye drops containing 0.05% doxycycline, these symptoms decrease in all cases to mild (Figure 2).

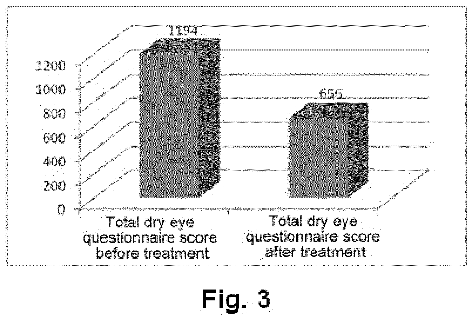

Figure 3 summarizes all the scores before and after treatment. A total score reduction can also be seen with respect to symptoms.

### Hyperemia according to the Cornea and Contact Lens Research Lab scale

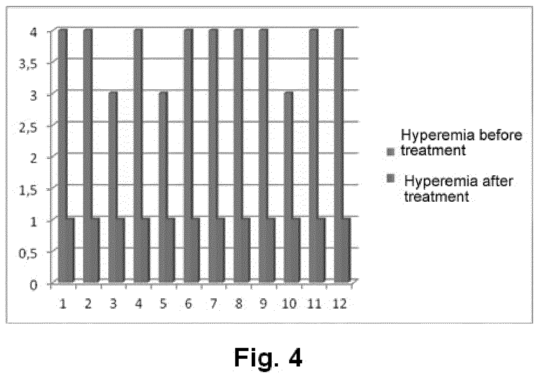

This scale allows including patients in this study based on hyperemia (redness of the eye) caused by this staphylococcal infection. The range thereof fluctuates according to the severity of hyperemia, between Grade I and Grade IV. As can be seen in the following graph, almost all the patients had severe grades of hyperemia before treatment. All the patients subsequently returned to minimum hyperemia levels (Figure 4).

In addition to these parameters, others such as the safety of the drug and its side effects were evaluated. In no case were there any withdrawals from treatment, epithelial keratitis or toxicities in the anterior pole of the eye resulting from this treatment, nor were there any significant increases in intraocular pressure.

The negative results obtained in all the staphylococcus cultures after treatment, the absence of side effects and the improvement of patients' symptoms led the authors of the invention to think of the long-term effectiveness that this drug may have in blepharitis, since this disease has no specific treatment at present. The results thereof are very promising and have prompted the authors of the invention to conduct a prospective clinical trial with a larger sample size.

### Example 2: Patient with blepharitis and glycemic decompensation.

A 72-year old male patient treated in the clinic for redness of the eye which he has been suffering for years. He received treatment provided by his primary care physician in the clinic but has not noticed any improvement, so he has requested specialized ophthalmology assistance. As a point of background interest, the patient suffers diabetes with poor glycemic control. Supervision of the fundus of the eye was performed in all check-ups due to diabetes.

First visit to the clinic on 5 March 2008: Upon examination of both eyes, signs of chronic anterior and posterior blepharitis, redness of the eye, telangiectasias, collarettes, secretions and mild-moderate level keratitis were confirmed. Treatment with tobramycin and dexamethasone in the form of eye drops and artificial tears was established at that time.

In this period of time and as a result of the blepharitis he suffered, infectious granules (styes) appeared on his eye lids and they had to be surgically removed (chalazion). The condition was then classified as severe blepharitis with a poor response to standard treatment.

As information of interest, the patient's blood glucose levels for the years 2007 and 2008 are provided below:

**Table 1. Blood glucose level of the patient for the years 2007-2008**

| Date | 1 Feb 2007 | 20 Feb 2008 | 29 Mar 2008 | 19 Jul 2008 | 18 Sept 2008 |
|---|---|---|---|---|---|
| Blood glucose (mg/dl) | 172 | 159 | 141 | 150 | 153 |

It must be borne in mind that for a fasting blood glucose test, a level between 70 and 100 milligrams per deciliter (mg/dl) is considered normal. For a random blood glucose test, a normal result will depend on the last time the patient ate. Most of the time, the blood glucose level will be below 125 mg/dl. This patient often showed figures up to twice what is considered normal in a diabetic process.

No diabetes-induced retinal lesions at that time.

Visit to the clinic on 21 November 2008: The patient mentioned that he had not noticed any significant improvement with the previous treatment, nor even had any worsening after withdrawing treatment. A new treatment with tobramycin and dexamethasone in the form of eye drops was started, reinforcing improvement of his symptoms with (topical anti-histamine) Zaditen eye drops.

No diabetes-induced retinal lesions at that time.

Visit to the clinic on 11 February 2009: In this visit to the clinic and due to the patient's symptoms worsening again with seborrheic, squamous blepharitis and new episodes of styes, a decision was made to apply a specific treatment with high doses of oral doxycycline for 20 days plus topical treatment with eye drops containing diclofenac and tobramycin with dexamethasone.

No diabetes-induced retinal lesions at that time.

Visit to the clinic on 12 May 2009: The patient again visited the clinic with worsening symptoms and mentioned that he did not take the complete treatment due to intolerance to oral doxycycline. He mentioned an episode of gastritis as a result of oral doxycycline and he therefore abandoned the treatment. Since doxycycline is particularly indicated for this pathology, a decision was then made to prescribe an individualized formula of eye drops containing 0.05% doxycycline with dexamethasone, and a topical treatment guideline for eye drops with dexamethasone and 0.05% doxycycline solution was indicated.

No diabetes-induced retinal lesions at that time.

Visit to the clinic on 15 June 2009: The patient visited the clinic to review this treatment, mentioning literally that "he is much better" (as reflected in the patient's history). The absence of telangiectasias, collarettes and inflammation as well as less redness were confirmed during examination. At that time and seeing the good result of therapy with eye drops containing 0.05% doxycycline with dexamethasone, treatment was prescribed and prescriptions for an additional 40 days were given due to the chronic nature of the process. In these future preparations, dexamethasone was excluded from the formulated product since it can cause glaucoma when topically applied in the form of eye drops for more than 25 days, so it was excluded and the patient was left with a topical preparation of 0.05% doxycycline eye drops.

No diabetes-induced retinal lesions at that time.

Visit to the clinic on 9 February 2010: After the previous cycle of topical doxycycline treatment, the patient remained asymptomatic although a new episode of seborrheic blepharitis appeared after withdrawing doxycycline when the prescriptions finished. A new treatment was prescribed with topical doxycycline until the new check-up in November and prescriptions were given.

No diabetes-induced retinal lesions at that time.

Visit to the clinic on 8 November 2010: The patient mentioned that he has continued with treatment because the symptoms of blepharitis reappeared when he abandoned treatment for a period of a few weeks, which irritates him; therefore he has continued with doxycycline eye drops indefinitely. The patient himself requested doxycycline eye drops in the pharmacy.

No diabetes-induced retinal lesions at that time.

Visit to the clinic on 1 August 2011: The patient visited the clinic for diabetes and blepharitis check-ups. He mentioned that he has been using doxycycline eye drops whenever the need arises depending on symptoms, but he was administrating the eye drops almost continuously because if he stops using it, discomfort and red eye come back.

No diabetes-induced retinal lesions at that time.

As information of interest, the patient's blood glucose levels for the years 2010, 2011 and 2012 are provided below:

**Table 2. Blood glucose level of the patient for the years 2010-2012**

| Date | 14 May 2010 | 14 Nov 2010 | 18 Nov 2010 | 20 Dec 2010 | 9 Sept 2011 | 22 Sept 2011 | 18 Sept 2012 |
|---|---|---|---|---|---|---|---|
| Blood glucose (mg/dl) | 146 | 232 | 170 | 175 | 201 | 219 | 147 |

There were no signs of diabetic retinopathy during the entire time this patient underwent doxycycline treatment both orally and in the form of eye drops, i.e., from 11 February 2009 to present.

In 2013 and given the improvement of his symptoms, he began to stop using eye drops containing doxycycline and to control his blood glucose levels.

Today, he shows no retinal alterations as a result of diabetes and his blood glucose levels are normal.

Based on the foregoing it can be concluded that the diabetes of the diabetic patient with high blood glucose levels and poor endocrine-metabolic control did not progress into diabetic retinopathy.

Likewise, based on the results obtained in the administration of doxycycline to this patient, the authors of the invention think that there is a cause and effect relationship between the administration of doxycycline and the absence of the onset of diabetic retinopathy despite the patient having a high blood glucose level for years, besides the curing of his blepharitis which remains inactive at present.

### Example 3: Composition of the eye drops containing doxycycline in liposomes

The eye drops used contain doxycycline in liposomes. These liposomes have been produced by means of the sonication method with subsequent extrusion. The liposomes have a final diameter of 200 nm. Fundamentally, the composition of these liposomes is based on soy lecithin with 90% phosphatidylcholine, and the liposomes are produced with purified water or physiological saline, and they will have a neutral or positive character, respectively, depending on this production. The final phospholipid concentration in the eye drops is therefore 5%.

Doxycycline is at a concentration of 0.05% within the liposome.

The use of antioxidants and/or stabilizer has been contemplated. Furthermore, it has been sterilized means of a filtration method using 0.22 µm filters.

### INVENTION 2

### Composition for the relief, improvement, prevention and/or treatment of dry eye syndrome

The present invention is comprised in the field of biology and medicine, and relates to the use of several plant species in the production of a medicament for ophthalmology therapy. Preferably, the present invention relates to the use of plants from the genera *Matricaria, Thymus* and *Aloe* in the production of a medicament for the relief, improvement, prevention and/or treatment of ocular pathologies, and more preferably for the treatment of dry eye syndrome.

### BACKGROUND OF THE INVENTION 2

Dry eye syndrome is an extremely common disease affecting millions of people. Its prevalence according to the environment can reach up to 35%, even reaching up to 75% in daily visits to clinics in Spain. The incidence tends to increase due to lifestyle changes, pollution, life expectancy, the use of screens, eye surgery and the use of contact lenses. It has a multifactorial origin and its symptoms include eye discomfort, reduced visual acuity and changes in relation to tears either due to deficiency or evaporation thereof, in addition to a greater susceptibility to infections. Dry eye is accompanied by an increased tear osmolarity and ocular surface inflammation. Various treatments have been proposed and they range from simple lubrication to the use of surgical or immunosuppressive means. According to the experience of the authors of the present invention, lubricants as a first line treatment are rather ineffective and do not bring about any anti-inflammatory, anti-infection or epithelization effect that the ingredients to be evaluated in the formula of the present invention do have.

From the clinical viewpoint, dry eye diseases included in the tear film dysfunction syndrome have various etiological causes, different combinations of anatomical and pathological involvements and several degrees of severity. A dacryology specialist must know these three parameters, quantify them and accordingly establish the appropriate treatment. Several classifications have been elaborated to that end. First, an etiological classification into age-related, hormonal, pharmacological, immunopathic, hyponutritional, dysgenetic, inflammatory, traumatic, neurodeprivative and tantalic causes, each of these groups including several variants. Second, a histopathological classification or ALMEN, an acronym for aquodeficiency, lipodeficiency, mucodeficiency, epitheliopathy and the involvement of other non-ocular exocrine glands. Third, a classification of severity into five grades: subclinical (symptoms in situations of overexposure), mild (common symptoms), moderate (reversible symptoms and signs), severe (irreversible symptoms and signs) and disabling (irreversible loss of sight due to corneal damage).

Dry eye syndrome can also be caused by Sjögren's syndrome. Sjögren's syndrome is an autoimmune disorder in which tear-producing cells are destroyed. The main symptoms of Sjögren's syndrome are dryness, burning and the feeling of there being a foreign body in the eye, being able to lead to blindness.

A wide range of treatments, i.e., lubricating eye drops ranging from common topical anti-inflammatory eye drops such as diclofenac, corticoids such as dexamethasone, to immunomodulators and immunosuppressants such as cyclosporin and tacrolimus, have been used for this pathology. All these drugs have side effects, are not effective for all cases and require preservatives in their solution, which do not help to provide stability to the ocular surface, requiring to continuously monitor the patient to whom these drugs have been administered in order to check for the absence of said side effects (Deveci and Kobak, 2014. Int. Ophthalmol. Jan 19). Lubricants such as carmellose and hyaluronate, without any anti-inflammatory, anti-infection or healing capacity, are often used. Washes with home-made chamomile preparations that may be safer and more effective than the simple use of lubricants are empirically known in ophthalmology. Combined with other active ingredients, an inexpensive effective product without chemical media and preservatives that are damaging to the ocular surface, reducing inflammation and red eye caused by this pathology with a multifactorial cause, could be developed.

### BRIEF DESCRIPTION OF THE INVENTION 2

A **first aspect** of the invention relates to a composition, hereinafter composition of the invention, comprising plant material from plants of the genera *Matricaria, Thymus* and *Aloe,* the extracts of said plants, or an active ingredient obtained from said plant material or the extracts.

In a preferred embodiment of this aspect, the composition of the invention further comprises a pharmaceutically acceptable vehicle. In another preferred embodiment of this aspect, the composition of the invention further comprises another active ingredient. In another more preferred embodiment, the composition of the invention is a pharmaceutical composition.

A **second aspect** of the invention relates to the composition of the invention for use as a medicament, or alternatively, to the use of the composition of the invention in the production of a medicament.

A **third aspect** of the invention relates to the composition of the invention for use in the relief, improvement, prevention and/or treatment of the dry eye syndrome, or alternatively, to the use of the composition of the invention in the production of a medicament for the relief, improvement, prevention and/or treatment of the dry eye syndrome.

A **fourth aspect** of the invention relates to the composition of the invention for use in the relief, improvement, prevention and treatment of Sjögren's syndrome, or alternatively, to the use of the composition of the invention in the production of a medicament for the treatment of Sjögren's syndrome.

A **fifth aspect** of the invention relates to a dosage form, hereinafter dosage form of the invention, comprising the pharmaceutical composition of the invention.

In a preferred embodiment of this aspect, the dosage form of the invention is selected from the list comprising: dressing, ointment, paste, cream, solution, suspension, emulsion, lotion, liniment, jelly, gel, foam, powder, or any combinations thereof. In another more preferred embodiment of this aspect, the dosage form of the invention is a solution, and even more preferably an ocular solution.

A **sixth aspect** of the invention relates to a method for producing a composition comprising the steps of:
a) mixing the plant material from plants of the genera *Matricaria, Thymus* and *Aloe,* the extracts of said plants, or an active ingredient obtained from said plant material or the extracts;
b) adjusting the pH.
   A preferred embodiment of this aspect of the invention further comprises:
c) sterilizing the solution.

In another preferred embodiment of this aspect of the invention, the mixing in a) is performed in water or a hydrate.

In another preferred embodiment of this aspect of the invention, the mixing in a) is performed in an anhydrous base.

In another preferred embodiment of this aspect of the invention, the proportion of a) is as follows: 1-48% *Matricaria,* 1-48% *Thymus* and 1-48% *Aloe.* More preferably, the proportion of a) is as follows: 3.3-20% *Matricaria,* 2.3-11.1 % *Thymus and* 4.4-22.3% *Aloe,* and even more preferably 7.5% *Matricaria,* 5% *Thymus and* 10% *Aloe.*

### DESCRIPTION OF THE DRAWINGS

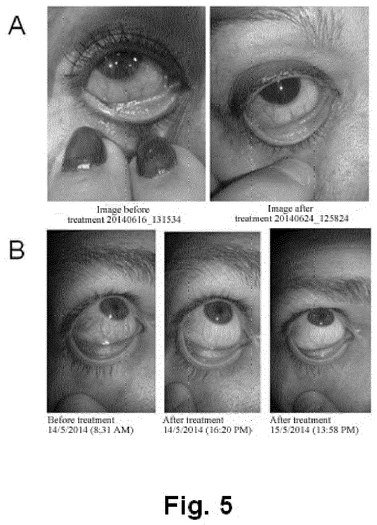

**Figure 5****. (A)** Hyperemia (redness of the eye) reduced after treatment in a case of dry eye syndrome associated with chronic treatment of antihypertensive eye drops with prostaglandins lasting for more than 10 years. Improvement was assessed 10 days after treatment in a case as severe as the one at hand. Patient from a cohort of 10 patients, which is the object of Example 4 of the invention: Treatment with *Aloe, Matricaria* and *Thymus* eye drops in patients with dry eye of any cause excluding Sjögren's syndrome.

**(B)** Improvement of hyperemia 8 and 24 hours after applying *Aloe, Matricaria* and *Thymus* eye drops in red eye resulting from dry syndrome associated with mild, blepharitic-type infection that went away without the administration of other anti-inflammatory eye drops or topical antibiotics. Patient extracted from Example 4 of the invention: Treatment in patients with involvements and pathologies of another type not related to dry eye syndrome: Blepharitis: 2 cases.

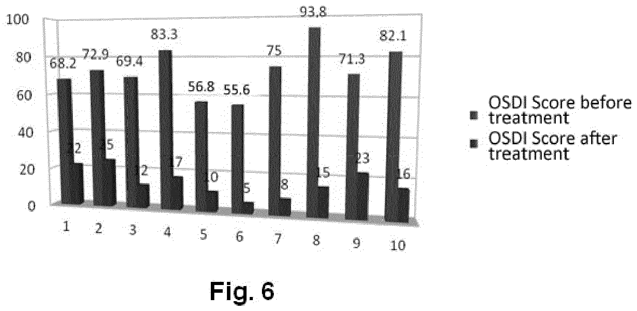

**Figure 6****.** OSDI (Ocular Surface Disease Index or ocular comfort test) questionnaire score before and after treatment (3 weeks). The higher the score, the larger the number and the greater the intensity of the symptoms derived from dry eye syndrome will be. A score of 0 to 100 is given. This figure summarizes the score of the OSDI test performed on a cohort of 10 patients which is the object of Example 4.

### DETAILED DESCRIPTION OF THE INVENTION 2

The authors of the present invention have developed a composition based on natural products with an anti-inflammatory and an anti-infection effect, in a correct proportion, which are safer and have fewer side effects than common topical anti-inflammatory drugs such as diclofenac or other immunomodulators and immunosuppressants, and these natural products in turn are more effective than the simple use of lubricants.

Therefore, a **first aspect** of the invention relates to a composition, hereinafter composition of the invention, comprising plant material from plants of the genera *Matricaria, Thymus* and *Aloe,* the extracts of said plants, or an active ingredient obtained from said plant material or the extracts.

In this specification, "plant material" is understood as either the aerial or subterranean parts of plants or other plant materials such as saps, resins, fatty oils, essential oils and any other substances of this nature, and combinations thereof, in crude form or in the form of plant preparations.

"Extracts" are concentrated preparations having a liquid, solid or intermediate consistency, usually obtained from a fresh, more preferably dried, plant material. For some preparations, the material to be extracted may require prior treatment, such as enzyme inactivation, grinding or degreasing, for example. The extracts are prepared by maceration, percolation or by other suitable validated methods using ethanol or another solvent. After the extraction, unwanted substances are eliminated, if required. Extraction methods are known by the person skilled in the art and are described, for example but without limitation, in the Royal Spanish Pharmacopeia or in the European Pharmacopeia. Extraction technologies include, among others, supercritical fluid extraction and other more conventional methods, such as steam extraction methods, solution (solid-liquid or liquid-liquid) extraction methods and centrifugation extraction methods.

In this specification, *"plant"* is understood as all the organisms which can be classified in the *kingdom Viridiplantae,* including green algae and land plants (Embryophyte).

The organisms of the genus *Matricaria belong to the Eukaryota superkingdom, Viridiplantae kingdom, Streptophyta phylum, Asterales order and Asteraceae family.*

More preferably, the organisms of the genus *Matricaria* are selected from the list consisting of: *Matricaria chamomilla, Matricaria discoidea* and *Matricaria matricarioides,* or any combinations thereof. Even more preferably, it is *Matricaria chamomilla* var. *recutita.*

The organisms of the genus *Thymus* belong to the *Eukaryota superkingdom, Viridiplantae kingdom, Streptophyta phylum, Lamiales order* and *Lamiaceae family.*

More preferably, the organisms of the genus *Thymus* are selected from the list consisting of: *Thymus alsinoides, Thymus amurensis, Thymus broussonetii, Thymus broussonetii subsp. hannonis, Thymus caespititius, Thymus daenensis, Thymus daenensis subsp. daenensis, Thymus dahuricus, Thymus haussknechtii, Thymus herba-barona, Thymus linearis, Thymus longicaulis, Thymus magnus, Thymus mandschuricus, Thymus marschallianus, Thymus mastichina, Thymus mongolicus, Thymus oenipontanus, Thymus oxyodontus, Thymus paronychioides, Thymus persicus, Thymus praecox, Thymus praecox subsp. polytrichus, Thymus proximus, Thymus pubescens, Thymus pulegioides, Thymus pulegioides var. pulegioides, Thymus quinquecostatus, Thymus quinquecostatus var. przewalskii, Thymus saturejoides, Thymus saturejoides subsp. commutatus, Thymus serpyllum, Thymus sipyleus, Thymus spinulosus, Thymus striatus, Thymus striatus var. acicularis, Thymus striatus var. ophioliticus, Thymus striatus var. striatus, Thymus trautvetteri, Thymus vulgaris, Thymus x citriodorus,* and *Thymus zygioides,* or any combinations thereof.

The organisms of the genus *Aloe belong to the Eukaryota superkingdom, Viridiplantae kingdom, Streptophyta phylum, Asparagales order* and *Xanthorrhoeaceae family.*

More preferably, the organisms of the genus *Aloe* are selected from the list consisting of: *Aloe acutissima, Aloe africana, Aloe albida, Aloe albiflora, Aloe alooides, Aloe angelica, Aloe anivoranoensis, Aloe arborescens, Aloe arborescens var. natalensis, Aloe arenicola, Aloe aristata, Aloe bakeri, Aloe bowiea, Aloe boylei, Aloe brevifolia, Aloe broomii, Aloe buhrii, Aloe bulbillifera, Aloe cameronii, Aloe cannellii, Aloe castanea, Aloe chabaudii, Aloe challisii, Aloe chortolirioides, Aloe comosa, Aloe compressa, Aloe compressa var. compressa, Aloe conifera, Aloe conifera var. conifera, Aloe craibii, Aloe cremnophila, Aloe dawei, Aloe deltoideodonta, Aloe dewinteri, Aloe dominella, Aloe dyeri, Aloe ecklonis, Aloe erinacea, Aloe excelsa, Aloe ferox, Aloe forbesii, Aloe fosteri, Aloe fouriei, Aloe glauca, Aloe globuligemma, Aloe gneissicola, Aloe greatheadii, Aloe greatheadii var. Davyana, Aloe haemanthifolia, Aloe haworthioides, Aloe hereroensis, Aloe hexapetala, Aloe humillis, Aloe inermis, Aloe integra, Aloe aff. Jacksonii, Aloe jucunda, Aloe juvenna, Aloe kniphofioides, Aloe kouebokkeveldensis. Aloe lavranosii, Aloe lineata, Aloe lineata var. Lineata, Aloe lineata var. Muirii, Aloe littoralis, Aloe lutescens, Aloe maculate, Aloe marlothii, Aloe melanacantha, Aloe microstigma, Aloe mitriformis, Aloe mitriformis subsp. Distans, Aloe modesta, Aloe morijensis, Aloe munchii, Aloe ngobitensis, Aloe niebuhriana, Aloe nubigena, Aloe nyeriensis, Aloe pearsonii, Aloe peckii, Aloe peglerae, Aloe pendens, Aloe penduliflora, Aloe perfoliata, Aloe perryi, Aloe petricola, Aloe pictifolia, Aloe pluridens, Aloe propagulifera, Aloe reynoldsii, Aloe rupestris, Aloe saundersiae, Aloe scobinifolia, Aloe sinkatana, Aloe somaliensis, Aloe somaliensis var. somaliensis, Aloe speciosa, Aloe spicata, Aloe striata, Aloe striata subsp. karasbergensis, Aloe striata subsp. komaggasensis, Aloe succotrina, Aloe suprafoliata, Aloe thompsoniae, Aloe thraskii, Aloe tomentosa, Aloe vaombe, Aloe variegate, Aloe vera, Aloe vera var. chinensis, Aloe verecunda, Aloe viguieri, Aloe vossii, Aloe vryheidensis, Aloe welwitschii, Aloe x spinosissima, Aloe zebrine,* or any combinations thereof. More preferably, it is *Aloe vera.*

In a preferred embodiment, other compounds which may or may not already be present can additionally be added to the composition of the invention, said compound being selected from the list consisting of: caricine, emodin, mannose phosphate, phytosterols, plant hormones, chamazulene and/or carvacrol.

Caricine is a chemical component stimulating the immune system that can be found in *Aloe vera.*

Emodin is an anthraquinone used for its laxative properties in pharmacological treatments which at present include severe pancreatitis.

Mannose phosphate is useful for promoting (speeding up) wound healing, *i.e.,* it has a healing effect.

Phytosterols or plant sterols (sterols from plants) are natural plant-based sterols. They are organic molecules that are part of the plant cell membrane, with a function similar to that of cholesterol in animal cell membranes. The main effect of phytosterols is their anti-inflammatory property so they are used in anti-aging products. In clinical studies, phytosterols have the property of improving damaged skin.

Plant hormones are substances produced by plant cells in specific locations in the plant and they regulate the physiological phenomena of plants. The main plant hormones that are known are: abscisic acid, auxins, cytokines, ethylene, gibberellins and brassinosteroids. There are other regulator compounds that could be considered: polyamines, oxylipins, salicylates, oligosaccharins, strigolactone and systemin. Specific plant hormones promote epithelization and cell growth in humans. Chamazulene is an aromatic chemical compound having a molecular formula of C₁₄H₁₆ found in a variety of plants, including in chamomile (*Matricaria chamomilla),* wormwood (*Artemisia absinthium),* and yarrow *(Achillea millefolium).* It is an azulene derivative biosynthesized from sesquiterpene matricin. Chamazulene has *in vivo* anti-inflammatory properties.

Carvacrol, or cymophenol, C₆H₃CH₃(OH)(C₃H₇), is a monoterpenoid phenol. It is found in essential oils such as oregano and thyme essential oils (5% and 75%) and others, and is responsible for the smell. This compound prevents infections by specific species of bacteria of the genera *Escherichia, Bacillus* or *Pseudomonas.*

In another preferred embodiment, the composition of the invention is a pharmaceutical composition. The compositions of the present invention can be formulated for administration to an animal, and more preferably to a mammal, including humans, in a range of forms known in the state of the art. In this sense, these compositions can be, without limitation, in a sterile aqueous solution or in biological fluids, such as serum. The aqueous solutions may or may not be buffered and they have additional active or inactive components. The additional components include salts for modulating ionic strength, preservatives including, but not limited to, antimicrobial agents, antioxidants, chelating agents, and the like, and nutrients including glucose, dextrose, vitamins and minerals. Alternatively, the compositions can be prepared for administration in solid form. The compositions can be combined with several inert vehicles or excipients, including but not limited to agglutinants such as microcrystalline cellulose, tragacanth gum, or gelatin; excipients such as starch or lactose; dispersing agents such as alginic acid or corn starch; lubricants such as magnesium stearate, glidants such as colloidal silicon dioxide; sweetening agents such as sucrose or saccharin; or aromatizing agents such as mint or methyl salicylate.

Therefore, in a preferred embodiment of this aspect of the invention the pharmaceutical composition can further comprise a pharmaceutically acceptable vehicle. In another preferred embodiment, the pharmaceutical composition further comprises excipients. In another preferred embodiment, the pharmaceutical composition can further comprise another active ingredient.

As it is used herein, the term *"active ingredient", "active substance", "pharmaceutically active substance"* or *"pharmaceutically active ingredient"* means any component that may provide a pharmacological activity or another different effect in the diagnosis, cure, mitigation, treatment, or prevention of a disease, or that affects the body structure or function of humans or other animals. The term includes those components that promote a chemical change in the production of the drug and are present in the drug in an envisaged modified form, providing the specific activity or effect.

In a preferred embodiment of this aspect, the composition of the invention further comprises a pharmaceutically acceptable vehicle. In another preferred embodiment of this aspect, the composition of the invention further comprises another active ingredient. In another more preferred embodiment, the composition of the invention is a pharmaceutical composition.

A **second aspect** of the invention relates to the composition of the invention for use as a medicament, or alternatively, to the use of the composition of the invention in the production of a medicament.

As it is used herein, the term *"medicament"* refers to any substance used for the prevention, diagnosis, relief, treatment or cure of diseases or their symptoms in humans and animals.

Herbal medicaments are defined in this specification as finished and labeled medicinal products the active ingredients of which consist of aerial or subterranean parts of plants or other plant materials or combinations thereof, in crude form or in the form of plant preparations. Herbal medicaments can also contain natural, organic or inorganic active ingredients that are not plant origin.

Plant preparations include ground or crushed plant materials, extracts, dyes, fatty or essential oils, saps obtained by pressing and preparations the production of which involves fractionation, purification or concentration. In a preferred embodiment of this aspect of the invention, the plant material is a plant preparation.

A **third aspect** of the invention relates to the composition of the invention for use in the relief, improvement, prevention and/or treatment of dry eye syndrome, or alternatively, to the use of the composition of the invention in the production of a medicament for the relief, improvement, prevention and/or treatment of dry eye syndrome.

Keratoconjunctivitis sicca (KCS), also called dry eye syndrome or keratitis sicca, is an ocular disease caused by dryness of the eye, which is in turn caused by any reduction in the production of tears or increased evaporation of the tear film. It is found in humans and some animals. KCS is the most common disease of the eye, affecting 5-6% of the population. The prevalence goes up to 6-9.8% in post-menopausal women (Schaumberg et al., 2003. Am J Ophthalmol 136 (2), 318-26), and goes as high as 34% in the elderly (Lin et al., 2005. Invest Ophthalmol Vis Sci 46 (5), 1593). The Latin phrase, *keratoconjunctivitis sicca* means "dryness of the (inflamed) cornea and conjunctiva".

A preferred embodiment of this aspect of the invention relates to the composition of the invention for use in the treatment of dry eye syndrome associated with Sjögren's syndrome, or alternatively, to the use of the composition of the invention in the production of a medicament for the treatment of dry eye syndrome associated with Sjögren's syndrome.

A **fourth aspect** of the invention relates to the composition of the invention for use in the relief, improvement, prevention and treatment of Sjögren's syndrome, or alternatively, to the use of the composition of the invention in the production of a medicament for the treatment of Sjögren's syndrome.

A **fifth aspect** of the invention relates to a dosage form, hereinafter dosage form of the invention, comprising the pharmaceutical composition of the invention.

In a preferred embodiment of this aspect, the dosage form of the invention is selected from the list comprising: dressing, ointment, paste, cream, solution, suspension, emulsion, lotion, liniment, jelly, gel, foam, powder, or any combinations thereof. In another more preferred embodiment of this aspect, the dosage form of the invention is a solution, and even more preferably, an ocular solution.

In this specification, *"dosage form"* is understood as the mixture of one or more active ingredients with or without additives having physical characteristics for suitable dosing, conservation, administration and bioavailability.

A *"dressing"* or *"patch"* is a dosage form consisting of a solid or semisolid form containing the active ingredient or ingredients and additives, spread on a piece of fabric, plastic or adhesive tape, acting as a support and protection, in addition to having an occlusive effect and macerating action which furthermore allows it to come into direct contact with skin and to soften with body temperature.

An *"unguent"* or *"ointment"* is a dosage form consisting of a preparation having a soft consistency containing the active ingredient or ingredients and additives incorporated to a suitable base giving it body and consistency. It is adhered and applied to the skin and mucous membranes. This base can be fat- or water-soluble, generally anhydrous or with a maximum of % of water. It is also referred to as hydrophilic unguent when it contains a base that can be washed away or removed with water.

A *"paste"* is a dosage form consisting of a semisolid form containing the active ingredient or ingredients and additives, made based on a high concentration of insoluble powders (20 to 50%), in weak abrasive or absorbent fatty or aqueous bases combined with soaps.

A *"cream"* is a dosage form consisting of a liquid or semisolid preparation containing the active ingredient or ingredients and additives required for obtaining an emulsion, generally an oil-in-water emulsion, with a water content greater than %.

A *"solution"* is a dosage form consisting of a transparent and homogenous liquid preparation obtained by dissolving the preparation or active ingredients and additives in water and is intended for external or internal use. In the case of injectable solutions for the eyes and ears, they must be sterile.

A *"suspension"* is a dosage form consisting of a dispersed system formed by two phases which contain the active ingredient or ingredients and additives. One of the phases, the continuous or external phase, is generally a liquid or a semisolid, and the dispersed or internal phase is made up of solids (active ingredients) that are insoluble but dispersible in the external phase. In the case of injectable suspensions, they must be sterile

An *"emulsion"* is a dosage form consisting of a heterogeneous system generally made up of two liquids not miscible with one another; in which the dispersed phase is formed by small globules distributed in the vehicle in which they are immiscible. The dispersed phase is also known as an internal phase and the dispersion medium is known as an external or continuous phase.

There are water-in-oil-type or oil-in-water-type emulsions and they can be presented as semisolids or liquids. The active ingredient or ingredients and additives can be in the external or internal phase.

A *"lotion"* is a dosage form that can be presented as a solution, suspension or emulsion, containing the active ingredient or ingredients and additives, and the dispersing agent of which is predominantly water.

A *"liniment"* is a dosage form consisting of an emulsion, solution or liquid presentation containing the active ingredient or ingredients and additives the vehicle of which is an aqueous, alcoholic or oily vehicle.

A *"jelly"* is a dosage form consisting of a semisolid colloid containing the active ingredient or ingredients and additives, the water-soluble base of which is generally made up of gums such as tragacanth gum, other bases used are: glycerin, pectin, alginates, boroglycerine compounds, synthetic derivatives or natural substances such as carboxymethyl cellulose.

A *"gel"* is a dosage form consisting of semisolid preparation containing the active ingredient or ingredients and additives, solids in a liquid which can be water, alcohol or oil, such that a network of particles trapped in the liquid phase is formed.

A *"foam"* is a dosage form consisting of a semisolid preparation formed by two phases: a liquid phase carrying the active ingredient or ingredients and additives, and another gaseous phase carrying propellant gas for the product to come out in the form of a cloud.

*"Powder"* is a dosage form consisting of a solid form containing the active ingredient or ingredients and additives that are finely ground and mixed to assure homogeneity.

A **sixth aspect** of the invention relates to a method for producing a composition comprising the steps of:
a) mixing the plant material from plants of the genera *Matricaria, Thymus* and *Aloe,* the extracts of said plants, or an active ingredient obtained from said plant material or from the extracts;
b) adjusting the pH.
   A preferred embodiment of this aspect of the invention further comprises:
c) sterilizing the solution.

In another preferred embodiment of this aspect of the invention, the mixing in a) is performed in water or a hydrate.

In another preferred embodiment of this aspect of the invention, the mixing in a) is performed in an anhydrous base.

In another preferred embodiment of this aspect of the invention, the proportion of a) is as follows: 1-48% *Matricaria,* 1-48% *Thymus* and 1-48% *Aloe.* More preferably, the proportion of a) is as follows: 3.3-20% *Matricaria,* 2.3-11.1 % *Thymus and* 4.4-22.3% *Aloe,* and even more preferably 7.5% *Matricaria,* 5% *Thymus and* 10% *Aloe.*

In another preferred embodiment of this aspect of the invention, the pH of b) is between 5 and 9, more preferably between 6 and 8, more preferably between 7 and 7.9, more preferably between 7.3 and 7.5, and even more preferably 7.4.

The sterilization process of solution c) can be carried out by any sterilization method known in the state of the art.

Throughout the description and claims the word "comprises" and variants thereof do not seek to exclude other technical features, additions, components or steps. For those skilled in the art, other objects, advantages and features of the invention will be inferred in part from the description and in part from the practice of the invention. The following examples and drawings are provided by way of illustration and do not seek to limit the present invention.

### EXAMPLES OF THE INVENTION 2

### Example 1: Preparation of the composition

### MATERIAL AND METHODS

Work was conducted in a clean room and with all the required measures to obtain a sterile product.

The amount of each element required for the formulation was calculated:

| | |
|---|---|
| a. Chamomile flower glycolic extract | 7.5% |
| b. Thyme glycolic extract | 5% |
| c. Pulp-free aloe vera gel | 10% |
| d. Water for injection, qsf | 10 ml |

The required water for injection (77.5%) is calculated. The extracts and Aloe vera gel are placed in a beaker. 80% of the required water for injection is added. pH is measured. pH is adjusted to 7.4 with 0.1 N NaOH, if required. The rest of the water for injection is added up to the required volume.

It is then filtered into opaque, already sterile vessels using a 0.22 µm filter.

A label is added which includes the composition, production date, expiration and any other element required for correctly identifying the staff preparing it and the user. It is kept in the refrigerator.

Detail of the functional active ingredients:

### • Chamomile flower glycolic extract:

i. Supplier: Fagron.
ii. Batch No. L13090187.
iii. INCI Composition: Propylene glycol, Aqua, *Chamomilla recutita.*
iv. Some physical specifications:
   1. Relative density: 1.04 g/ml.
   2. Refractive index: 1.393.
   3. Heavy metals: < 20 ppm.
   4. pH: 5.8

### • Thyme glycolic extract:

v. Supplier: Metalpharmaceutical. (A.C.E.F.).
vi. Batch No. K2608001
vii. INCI composition: Propylene glycol, Water, *Thymus vulgaris* flower/leaf extract
viii. Some hysical specifications:
   1. Drug/Extract concentration: 1:2
   2. pH (10% solution): 4.8
   3. Heavy metals: < 20 ppm
   4. Preservatives: methyl, ethyl, propyl, butyl - paraben in phenoxyethanol, 0.2%

### • Pulp-free Aloe vera gel:

ix. Supplier: Acofarma.
x. Batch: 140136.
xi. Some physical specifications:
   1. Density: 0.997 g/ml.
   2. Refractive index: 1.3341.
   3. Anthraquinones: < 0.1%.
   4. Parenchyma/extract ratio: 1:1
   5. Preservatives: potassium sorbate, sodium benzoate.

### Example 2: Case 1 of dry eye syndrome in the context of Sjögren's syndrome:

Patient was referred by the Rheumatology Department of Hospital Reina Sofía, Cordoba, for syndrome due to discomfort, itching and sporadic difficulty in seeing to be evaluated for autoimmune dry syndrome and treatment. Patient was unsuccessfully treated with topical corticoids and lubricants. These visits took place on the following dates: 17/06/2014 and 07/07/2014.

Patient was diagnosed in these visits with dry eye syndrome contrasted with the original syndrome diagnosis. In the visit to the clinic on 17/06/2014, treatment was implemented with eye drops containing aloe, thymus and chamomile after several treatments which did not ease symptoms. Patient has a checkup on 07/07/2014, and an improvement of symptoms is seen both subjectively and objectively. A table containing the main parameters collected in the visit prior to establishing treatment with the eye drops object of this patent document and after 12 days of treatment with said treatment is shown below.

**Table 1. Data on ophthalmological tests in case 1 of Sjögren's syndrome.**

| | Visit on 17/06/2014 | Visit on 07/07/2014 |
|---|---|---|
| Schirmer, right eye | 13 | 30 |
| Schirmer, left eye | 11 | 25 |
| Break up time | 5 | >9 |
| Hyperemia scale | Grade II | Grade II |
| OSDI Test | 95.83 | 4.54 |

### Example 3: Case 2 of dry eye syndrome in the context of Sjögren's syndrome:

Patient came on 04/08/2014 referred by the Rheumatology Department for discomfort and itchy eyes. She has a prior diagnosis of syndrome and has been treated, without showing any improvement of symptoms, with lubricants, corticoids, autologous serum and cyclosporin. Visits were on the following dates: 04/08/2014 and 01/09/2014.

In the visit to the clinic on 04/08/2014, treatment was established months ago with aloe, thymus and chamomile eye drops after treatment with artificial tears, corticoids, autologous serum and cyclosporin in a private center that have not eased the symptoms. In that center, patient was proposed treatment with growth factors, which he refused. Patient is examined on 01/09/2014, and an improvement of symptoms is seen both subjectively and objectively. A table containing the main parameters collected in the visit prior to establishing treatment with the eye drops object of this patent document and after treatment with aloe, thymus and matricaria eye drops from that visit.

**Table 2. Data on tests in case 2 of Sjögren's syndrome.**

| | Visit on 04/08/2014 | Visit on 01/09/2014 |
|---|---|---|
| Schirmer, right eye | 8 | 8 |
| Schirmer, left eye | 8 | 10 |
| Break up time | 3 | 8 |
| Hyperemia scale | Grade III | Grade I |
| OSDI Test | 100 | 66.66 |

### Example 4: Treatment with aloe, matricaria and thymus eye drops in patients with dry eye of any origin excluding Sjögren's syndrome, and results of the preparation in other pathologies excluding dry eye syndrome

In patients with dry eye syndrome the standard treatment of which did not ease symptoms and treatment began with aloe, thymus and matricaria eye drops.

A group of 10 patients (n=10) were treated who met the following criteria:
- Adult population: mean adult age of 66 years old (3 men and 7 women).
- Patients diagnosed with dry eye symptoms (excluding syndrome).
- OSDI (Ocular Surface Disease Index©) score: prior mean 72.84.

Treatment with eye drops containing the preparation was carried out for 21 days. The photographs in Figure 5 belong to one of the 10 patients.

### RESULTS - OSDI

The OSDI© (Ocular Surface Disease Index©) score: evaluates on a scale of 0 to 100 the degree of involvement of the patient, where 100 is the most severe stage:
- Before the study, the results indicated a mean of 72.84 for this indicator.
- After the test, mean values dropped 75% to 15.3 being a significant improvement in 100% of the cases.

See the results in Figure 6.

In patients with disorders and pathologies of another type not related to dry eye syndrome:
- Recurring corneal ulcers: 2 cases.
- Corneal ulcers due to active VHZ (herpes): 2 cases.
- Blepharitis: 2 cases.

Several conclusions can be drawn from this study and are summarized in Table 3:

**Table 3. Results of applying aloe, thymus, matricaria eye drops in other pathologies not associated with dry eye syndrome.**

| **Pathology** | **Improvement** |
|---|---|
| Ulcers due to VHZ | Complete closure without stromal inflammatory infiltration. |
| Recurring ulcers | Complete closure-active epithelization from the start. |
| Blepharitis | Reduced range of the *tear corneal lab* from grade 4 to grade 1 (normal). No signs of infection. 3-month absence of symptoms maintained. |

The following can be cited as the main achievements of the use of this treatment: strength similar to cortisone, astringent, anti-inflammatory, reduces hyperemia to normal levels in 100% of cases, enormous epithelization, bacteriostatic and antimycotic effect, and absence of pathological side effects.

### CLAUSES

1. A composition comprising plant material from plants of the genera *Matricaria, Thymus* and *Aloe,* the extracts of said plants, or an active ingredient obtained from said plant material or from the extracts.
2. The composition according to the preceding claim, further comprising a pharmaceutically acceptable vehicle.
3. The composition according to any of claims 1-2, further comprising another active ingredient.
4. The composition according to any of claims 1-3, which is a pharmaceutical composition.
5. Use of the composition according to any of claims 1-4 in the production of a medicament.
6. Use of the composition of the invention in the production of a medicament for the relief, improvement, prevention and treatment of dry eye syndrome.
7. Use of the composition of the invention in the production of a medicament for the treatment of the syndrome.
8. A dosage form comprising the composition according to any of claims 1-4.
9. The dosage form according to the preceding claim selected from the list comprising: dressing, ointment, paste, cream, solution, suspension, emulsion, lotion, liniment, jelly, gel, foam, powder, or any combinations thereof. In another more preferred embodiment of this aspect, the dosage form of the invention is a solution, and even more preferably, an ocular solution.
10. The dosage form according to any of claims 9-10 which is a solution.
11. The dosage form according to the preceding claim which is an ophthalmic solution.
12. A method for producing an ophthalmic solution comprising the following steps:
   a) mixing the plant material from plants of the genera *Matricaria, Thymus* and *Aloe,* the extracts of said plants, or an active ingredient obtained from said plant material or from the extracts;
   b) adjusting the pH.
13. The method according to the preceding claim, further comprising:
   c) sterilizing the solution.
14. The method according to claims 12-13, wherein the mixing in a) is performed in water or a hydrate.
15. The method according to claims 12-14, wherein the mixing in a) is performed in an anhydrous base.
16. The method according to claims 12-15, wherein the proportion of a) is as follows: 7.5% *Matricaria,* 5% *Thymus* and 10% *Aloe.*

## Claims

1. Use of a composition comprising doxycycline or any of its pharmaceutically acceptable salts, esters, tautomers, solvates and hydrates, or any combinations thereof, in the production of a medicament for topical administration for the prevention, improvement and/or treatment of ocular pathologies.

2. The use of a composition according to the preceding claim, wherein the ocular pathology is selected from: blepharitis, microthrombosis in the anterior pole and chorioretinal vascular disorders, or any combinations thereof.

3. The use of a composition according to any of claims 1-2, wherein doxycycline is included in liposomes.

4. The use of a composition according to any of claims 1-3, wherein the liposomes have a cationic character.

5. The use of a composition according to any of claims 1-4, wherein the liposomes have a neutral character.

6. The use of a composition according to any of claims 1-5, wherein the composition has a concentration between 0.5 and 50% liposomes.

7. The use of a composition according to any of claims 1-6, wherein the composition has a concentration between 1 and 25% liposomes.

8. The use of a composition according to any of claims 1-7, wherein the composition has a concentration between 2 and 10% of liposomes.

9. The use of a composition according to any of claims 1-8, wherein the composition has a concentration between 4 and 6% of liposomes.

10. The use of a composition according to any of claims 1-9, wherein the composition has a concentration of about 5% of liposomes.

11. The use of a composition according to any of claims 1-10, wherein the liposomes are composed of lecithin.

12. The use of a composition according to any of claims 1-11, wherein the liposomes are composed of lecithin with 50 to 99% of phosphatidylcholine.

13. The use of a composition according to any of claims 1-12, wherein the liposomes are composed of lecithin with 70 to 95% of phosphatidylcholine.

14. The use of a composition according to any of claims 1-13, wherein the liposomes are composed of lecithin with about 90% of phosphatidylcholine.

15. The use of a composition according to any of claims 1-14, wherein the liposomes are stabilized in an ionic solution.

16. The use of a composition according to any of claims 1-15, wherein the liposomes are stabilized with phosphate buffer.

17. The usev of a composition according to claim 16, wherein the pH of the phosphate buffer is adjusted to a value between 4 and 10.

18. The use of a composition according to any of claims 16-17, wherein the pH of the phosphate buffer is adjusted to a value between 5 and 9.

19. The use of a composition according to any of claims 16-18, wherein the pH of the phosphate buffer is adjusted to a value between 6 and 8.

20. The use of a composition according to any of claims 1-14, wherein the liposomes are stabilized in a non-ionic solution.

21. The use of a composition according to claim 20, wherein the non-ionic solution is selected from a mannitol solution and a glucose solution.

22. The use of a composition according to claim 21, wherein the non-ionic solution is a mannitol solution.

23. The use of a composition according to claim 22, wherein the mannitol solution has a concentration between 1 and 20% mannitol.

24. The use of a composition according to claim 22, wherein the mannitol solution has a concentration between 2.5 and 10% mannitol.

25. The use of a composition according to claim 22, wherein the mannitol solution has a concentration of about 5% mannitol.

26. The use of a composition according to any of claims 20-25, wherein the pH of the solution is adjusted to a value between 3 and 9.

27. The use of a composition according to any of claims 20-26, wherein the pH of the solution is adjusted to a value between 4 and 8.

28. The use of a composition according to any of claims 20-27, wherein the pH of the solution is adjusted to a value between 5 and 7.

29. The use of a composition according to any of claims 1-28, wherein the liposomes have a final size between 20 and 1000 nm.

30. The use of a composition according to any of claims 1-29, wherein the liposomes have a final size between 50 and 500 nm.

31. The use of a composition according to any of claims 1-30, wherein the liposomes have a final size between 100 and 300 nm.

32. The use of a composition according to any of claims 1-31, wherein the liposomes have a final size of about 200 nm.

33. The use of a composition according to any of claims 1-32, wherein the composition has a doxycycline concentration between 0.01 and 5%.

34. The use of a composition according to any of claims 1-33, wherein the composition has a doxycycline concentration between 0.025 and 1%.

35. The use of a composition according to any of claims 1-34, wherein the composition has a doxycycline concentration between 0.04 and 0.06%.

36. The use of a composition according to any of claims 1-35, wherein the composition has a doxycycline concentration of about 0.05%.

37. The use of a composition according to any of claims 1-36, wherein the composition is a pharmaceutical composition.

38. The use of a composition according to any of claims 1-37, wherein the composition further comprises a pharmaceutically acceptable vehicle.

39. The use of a composition according to any of claims 1-38, wherein the composition further comprises another active ingredient.

40. The use of a composition according to any of claims 1-39, wherein the liposomes comprise antioxidants.

41. The use of a composition according to any of claims 1-40, wherein the liposomes comprise stabilizers.

42. The use of a composition according to any of claims 1-41, wherein the composition is sterilized.

43. A dosage form comprising a composition according to any of claims 1-42.

44. The dosage form according to the preceding claim which is selected from the list consisting of: dressing, ointment, paste, cream, solution, suspension, emulsion, lotion, liniment, jelly, gel, foam, powder, or any combinations thereof.

45. The dosage form according to any of claims 43-44, which is a solution.

46. The dosage form according to claim 45, wherein the solution is an ophthalmic solution.

47. Use of a dosage form according to any of claims 43-46 in the production of a medicament for topical administration for the prevention, improvement and/or treatment of ocular pathologies.

48. The use of a composition according to the preceding claim, wherein the ocular pathology is selected from: blepharitis, microthrombosis in the anterior pole and chorioretinal vascular disorders, or any combinations thereof.
